Europäisches Patentamt

European Patent Office

Office européen des brevets

(19)

(11) EP 1 355 154 A2

# (12) EUROPEAN PATENT APPLICATION

(43) Date of publication:
22.10.2003 Bulletin 2003/43

(51) Int Cl.⁷: **G01N 33/53**, C08B 37/00

(21) Application number: 03007314.2

(22) Date of filing: 31.03.2003

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **01.04.2002 JP 2002098989**

(71) Applicant: **Kyokuto Pharmaceutical Industrial Co. Ltd**
**Chuo-Ku, Tokyo 103-0024 (JP)**

(72) Inventors:
• **Miwa, Akinari, Kyokuto Pharm Ind. Co., Ltd.**
**Takahagi-shi, Ibaraki 318-0004 (JP)**

• **Araki, Hajime, Kyokuto Pharm Ind. Co., Ltd.**
**Takahagi-shi, Ibaraki 318-0004 (JP)**
• **Yamagishi, Yoshio, Kyokuto Pharm Ind. Co., Ltd.**
**Takahagi-shi, Ibaraki 318-0004 (JP)**
• **Minakawa, Kenji, Kyokuto Pharm Ind. Co., Ltd.**
**Takahagi-shi, Ibaraki 318-0004 (JP)**

(74) Representative: **Grünecker, Kinkeldey, Stockmair & Schwanhäusser Anwaltssozietät**
**Maximilianstrasse 58**
**80538 München (DE)**

## (54) Accelerator for agglutination reactions, reagent for biochemical assays, and biochemical assay method

(57) The object of the present invention is to provide an accelerator for agglutination reactions that is suitable to use in biochemical agglutination determination systems (e.g., immune agglutination determination systems), shows a strong accelerative function to agglutination reactions, does not tend to increase viscosities of its aqueous solutions, shows good reproducibility and precision in determination results, and can be readily handled.

The object can be attained by using an accelerator consisting essentially of at least one member selected from the group consisting of alginic acid and alginates.

The accelerator can be applied to reagents for biochemical assays (e.g., immunoassays), kits for biochemical assays, and biochemical assay methods.

Further, the present invention provides use of at least one member selected from the group consisting of alginic acid and alginates as an accelerator for agglutination reactions.

Figure 1

Legend:
- sodium alginate 0.1%
- sodium alginate 0.08%
- sodium alginate 0.07%
- sodium alginate 0.05%
- polyvinylpyrrolidone
- polyethylene glycol 500,000

Y-axis: ΔAbs. ×10000

X-axis: Conc. of antibody against syphilis surface antigen in standard serum (Unit: T.U.)

## Description

<u>Field of the Invention</u>

The present invention relates to an accelerator for biochemical assays (e.g., immunoassays) based on a principle of an agglutination reaction, a reagent for biochemical assays comprising the accelerator, a kit for a biochemical assay comprising the reagent, a biochemical assay method in which the accelerator is used, and use of alginic acid and/or an alginate as the accelerator.

<u>Background Art</u>

Immunoassays, in which a specific antigen or antibody is detected by using an immune agglutination reaction between an antigen and an antibody against it, have been widely conducted in various clinical diagnostic tests, biochemical research, and the like. In immunoassays, commonly, an antibody against an antigen that is a target to be detected or an antigen of an antibody that is a target to be detected is brought into contact with a specimen that may contain the target, and the amount of immune agglutinations is determined which have been formed in a reaction mixture as a result of an antigen-antibody reaction when the target is present in the specimen.

The antibody against an antigen or the antigen of an antibody may be supported on carriers. If the carriers are used, the immunoassay can give higher performance than the case where the carriers are not used. Thus, immunoassays in which carriers are used (for example, latex agglutination tests in which polystyrene beads are used as carriers) have been popularly conducted.

The amount of agglutinations is generally determined by nephelometry in which the intensity of scattered light is measured because the scattering of light occurs by the presence of agglutination or by turbidimetry in which the ratio of the intensity of transmitted light to that of incident light is measured because the transmission of light is obstructed by the agglutinations. Both of the nephelometry and turbidimetry have been commonly used although each has good and bad points.

While, in immunoassays, to improve the sensitivity and precision in determination, to facilitate an antigen-antibody reaction, and to stabilize agglutinations, various polymeric compounds such as polyethylene glycol, pulluran, polyvinyl pyrrolidone, and carboxymethyl cellulose are commonly used as accelerators. For example, Japanese Patent Early-publication No. H.05-180838 discloses using pulluran or polyvinyl pyrrolidone. Japanese Patent Early-publication No. H.10-282100 discloses using a surfactant having a molecular weight of 500-2,000 and an HLB of 25-80 (e.g., CHAPS, BIGCHAP, and digitonin). Japanese Patent Early-publication No. H.10-282101 discloses using a polymer having a molecular weight of 1,000-1,500,000 and being composed of a monomer having an HLB of 9-20 (e.g., polyvinyl pyrrolidone, polyethyleneimine, and polyethylene glycol). Japanese Patent Early-publication No. H.10-282100 discloses using a surfactant having a molecular weight of 500-2,000 and a steroid ring as its main skeleton or framing structure (e.g., CHAPS, BIGCHAP, and cycosaponin). Japanese Patent Early-publication No. 2001-74742 discloses as accelerators for agglutination reactions polyvinyl pyrrolidone, polyethyleneimine, polyethylene glycol, CHAPS, BIGCHAP, digitonin, saponin, pulluran, and poly(glycosyl ethylmethacrylate).

However, they may increase the viscosity of a reagent or a reaction mixture. Some of them may also facilitate non-specific reactions. Thus, there are drawbacks in uses of the above polymeric compounds in determination systems based on the principle of immune agglutination.

<u>Summary</u>

An object of the present invention is to provide an accelerator for agglutination reactions that is suitable to use in biochemical agglutination determination systems, shows a strong accelerative function to agglutination reactions, does not tend to increase viscosities of its aqueous solutions such as reagents and reaction mixtures, shows good reproducibility and precision in determination results, and can be readily handled, especially one that can contribute to realize in latex agglutination determination systems a high sensitivity and a determination or quantitative analysis in a wide range.

Another object of the present invention is to provide a reagent for biochemical assays that comprises the accelerator, a kit for a biochemical assay comprising the reagent, and a biochemical assay method that uses the accelerator or the reagent.

Another object of the present invention is to provide use of alginic acid and/or an alginate as the accelerator.

The present inventors have extremely studied to search an accelerator for agglutination reactions, by which the above objects can be attained. As a result they have found that alginic acid and alginates (e.g., sodium alginate and propylene glycol alginate) are excellent accelerators. Thus, they have accomplished the present invention.

Namely, the present invention provides an accelerator for agglutination reactions consisting essentially of at least one member selected from the group consisting of alginic acid and alginates.

Also, the present invention provides a reagent for biochemical assays comprising at least one member selected from the group consisting of alginic acid and alginates.

The reagent of the present invention includes the following embodiments alone or in combination of two or more of them:

a) the biochemical assays are immunoassays,

b) the reagent may be a diluent for specimens,

c) the reagent may further comprise a compound that can specifically bond to a target to be detected,

d) in the embodiment c), (1) the compound is an antibody and the target is an antigen of the antibody or (2) the compound is an antigen and the target is an antibody against the antigen,

e) in the embodiment c), (1) the compound is avidin and the target is biotin,

f) in the embodiment c), the reagent further comprises carriers on which the compound is supported, and

g) the reagent is one that is used in an immune agglutination reaction.

Further, the present invention provides a kit for a biochemical assay comprising the above reagent according to the present invention (hereafter "reagent (x)"), and at least one member selected from the group consisting of a reagent (y) other than the reagent (x), a container, and an instruction for use.

The kit may be used for an immunoassay.

Furthermore, the present invention provides a biochemical assay method comprising a step of bringing a compound that can specifically bond to a target to be detected into contact with a specimen that may contain the target in the presence of at least one member selected from the group consisting of alginic acid and alginates and a step of determining the amount of agglutinations that have been made by the specific reaction between the compound and the target.

In the biochemical assay method, (1) the compound may be an antibody and the target may be an antigen of the antibody or (2) the compound may be an antigen and the target may be an antibody against the antigen, and the specific reaction may be an antigen-antibody reaction.

Moreover, the present invention provides use of at least one member selected from the group consisting of alginic acid and alginates as an accelerator for agglutination reactions.

Brief Description of the Drawings

Figure 1 is a graph where accelerative effects of an immune agglutination reaction by various accelerators are compared in a latex agglutination determination system.

Figure 2 is a graph where accelerative effects of an immune agglutination reaction by diluents for specimens containing sodium alginate in various concentrations are compared in a latex agglutination determination system.

Detailed Description

Hereafter, the present invention will be specifically explained.

Examples of the agglutination reactions include immune agglutination reactions. Also, examples of the biochemical assays include immunoassays. Thus, hereafter we will explain the present invention with referring to immune agglutination reactions and immunoassays. However, the applications of the present invention are not limited to immune agglutination reactions and immunoassays. Agglutination reactions and biochemical assays in which a specific bonding or binding reaction between two compounds (e.g., avidin and biotin) is used are also included within the scope of the present invention.

1. Accelerator for Agglutination Reactions

In the present invention, an accelerator means a compound that can facilitate or help to occur agglutination reactions. The accelerator of the present invention consists essentially of at least one member selected from the group consisting of alginic acid and alginates. In this specification, alginic acid and alginates may be comprehensively called as "alginic type polymers." Alginates include alginic acid salts such as sodium alginate, calcium alginate, magnesium alginate, and ammonium alginate, and alginic esters such as propylene glycol alginate.

Alginic acid is a structural polysaccharide of *Phaeophyta* and has a high molecular structure of a straight chain in which D-mannuronic acid molecules are linked through β-1,4-linkage.

Among alginic type polymers, sodium alginate is desirable in view of storage stability and solubility. Sodium alginate is water-soluble, white or pale yellow powder. It is usually used as an emulsifier, an stabilizer, a water-absorbable polymer, or the like.

Among alginic type polymers, propylene glycol alginate is also desirable. This is because it readily dissolves in cold water, hot water, and acidic solutions, and does not precipitate in the presence of calcium, metal salts, etc.

In the present invention, alginic type polymers may be used alone or in combination of two or more of them.

The mechanism by which alginic type polymers facilitate agglutination reactions is thought as follows:

If in a colloidal solution colloidal particles such as latex particles come close to each other, polymers that are dissolved in the colloidal solution and take each a finite space are cleared away from spaces between the particles. Namely, the so-called depletion effect occurs. In the area where the depletion effect occurs, the colloidal particles are concentrated. Therefore, the frequency of collision of those particles is increased. While, target compounds to be detected bond to compounds that can specifically bond to the target compounds and that have been bonded to the surfaces of the colloidal particles, and thus immune complexes each comprising a colloidal particle are formed. Because the frequency of collision of the colloidal particles is increased, the complexes also collide with each other in a high frequency. Therefore, the target compounds in the complexes can bond to the compounds in other complexes in a high frequency. As a result, large complexes, namely, agglutinations, are formed.

More specifically, there is an area where antibody-bonded colloidal particles are concentrated by the depletion effect. If antigen molecules are put into the area, they bond to the antibodies and thus immune complexes each comprising an antigen molecule(s) and an antibody-bonded colloidal particle are formed. These complexes collide with each other in a high frequency. Therefore, the antigen molecules in the complexes can bond to the antibodies in other complexes in a high frequency. As a result, large immune agglutinations are formed. Thus, the immune agglutination may be facilitated.

The alginic type polymers have various molecular weights. Therefore, their aqueous solutions have various viscosities according to their molecular weights. The grades of commercial products of alginic type polymers are often specified by the viscosities (e.g., 100-150 cp, 300-400 cp, and 500-600 cp [1 w/v %, at 20°C]) that their aqueous solutions have. In the present invention, commercial products of all grades can be used. However, from the view points of handling and reproducibility, alginic type polymers, of which aqueous solution shows lower viscosities, are excellent. Thus, those, of which 1 w/v % aqueous solution shows a viscosity of 300 cp or less at 20°C, are desirable (more desirably 200 cp or less and most desirably 100-150 cp).

The accelerator of the present invention may be a composition. The composition may contain, as additives that do not adversely affect the functions of the alginic type polymers, a pH buffer agent, a salt, bovine serum albumin (BSA), a surfactant, and the like.

The form of the accelerator of the present invention is not limited. One example of the form is a powdery one. If the accelerator has a powdery form, it is usually used by dissolving in an aqueous medium. The aqueous medium may be one that has been usually used in the biochemical field. Examples of it include deionized water and various pH buffers such as phosphate buffers, glycine buffers, Good's buffers, Tris buffers, and ammonium salt buffers. Among them, phosphate buffers, glycine buffers, and Good's buffers are preferably used.

An accelerator composition of the present invention may have a form of an aqueous solution. The aqueous solution comprises, as essential components, an alginic type polymer and deionized water. It may comprise, in addition to the essential components, at least one member selected from the group consisting of a pH buffer agent, a salt, bovine serum albumin (BSA), and a surfactant. The accelerator composition may be the reagent for biochemical assays (e.g., immunoassays) according to the present invention.

The concentration of the alginic type polymer in the aqueous solution is preferably 0.001-1 w/v % from the view point of solubility and is more preferably 0.05- 1w/v % from the view point of convenience in use.

The accelerator of the present invention shows a sufficient effect even if it is used in a small amount. In a liquid where an agglutination reaction would occur, i.e., in a reaction mixture comprising various reagents and a specimen, the final concentration of the alginic type polymer is preferably 0.001-1 w/v %, more preferably 0.01-0.5 w/v %, still more preferably 0.01-0.3 w/v %, and most preferably 0.03-0.1 w/v %. If the concentration is too low, it may be difficult to obtain a sufficient effect about the facilitation of the agglutination reaction. If the concentration is too high, the viscosity may come to be too high, and thus the precision of determination and easiness of handling may be decreased.

2. Reagent for Biochemical Assays

The reagent for biochemical assays of the present invention comprises at least one member selected from the group consisting of alginic acid and alginates. The reagent may be a diluent for specimens. The reagent may further comprise a compound that can specifically bond to a target to be detected. The compound may be an antibody against an antigen that is a target to be detected or an antigen of an antibody that is a target to be detected. Thus, the reagent of the present invention may be a mixture of a reagent that has been used in, e.g., immunoassays and an alginic type polymer.

In biochemical assays such as immunoassays, various biological liquid sample such as serum and plasma is usually diluted with a liquid. The liquid for dilution is usually an aqueous solution that comprises at least one member selected from the group consisting of a pH buffer agent, a protein such as albumin or globulin, an amino acid, a surfactant and a salt. To prepare the reagent of the present invention, an alginic type polymer may be added to the liquid for dilution. The concentration of the alginic type polymer in the diluent for specimens, i.e., in the reagent of the present invention, is preferably 0.001-1 w/v % from the view point of solubility.

Another example of reagents that have been used in immune agglutination methods is a reagent hereafter "agglutination reagent") comprising a compound that reacts with a target to be detected by an antigen-antibody reaction. The agglutination reagent is usually a liquid comprising an antigen or antibody or a suspension comprising insoluble carriers on which an antigen or antibody is sensitized. To the agglutination reagent an alginic type polymer may be added. Thus, the reagent of the present invention may be prepared.

The target to be detected in immunoassays based on a principle of immune agglutination is an antigen or an antibody. Also, the compound that are contained in the agglutination reagent for immunoassays is an antibody or an antigen.

Examples of the antigen include receptors, enzymes, blood proteins such as carcinoembryonic proteins, and infectious disease-related antigens such as hepatitis B virus, hepatitis C virus, pathogen of syphilis (i.e., *Treponema pallidum*), human immunodeficiency virus, and pathogenic *Escherichia coli*. However, antigens in the present invention are not limited to those listed above. All compounds against which antibodies can be formed are antigens. Thus, in some cases antibodies may play as antigens. Further, as long as an antigenic determinant exists, a fragments, subunits, and compounds having the fragment or subunit may be antigens.

In this specification, the term "antibody" means compounds that bond to or bind with a specific antigen by an immunological reaction. Namely, as long as it can bind with a specific antigen, fragments of antibodies, for example, may also be called as "antibodies." Examples of the antibodies in the present invention include antibodies against the above-listed antigens. The antibodies may be polyclonal ones or monoclonal ones.

Thus, one example of the agglutination reagents comprises, as an essential component, an antibody when the target to be detected is an antigen or an antigen when the target to be detected is an antibody. Specifically, if the target is an antibody against *Helicobacter pylori*, the agglutination reagent comprises an antigen of *Helicobacter pylori*.

Other example of the agglutination reagents comprises avidin as an essential component, and is used to detect biotin.

As mentioned above, the agglutination reagent and the reagent for biochemical assays of the present invention may comprise insoluble carriers on which a compound that can specifically bond to a target, e.g., an antigen or antibody, is supported. The insoluble carriers are, in other words, sensitized or coated with the compound. Namely, the compound is immobilized on the carriers. As the carrier, insoluble particles or particulates having a diameter of about 0.1-0.5 micrometer are usually used. Specifically, the insoluble particles or particulates may be erythrocyte, carbon powder, bentonite, kaolin, micelle of lecithin, gelatin particles, polystyrene particles, or the like. Among these insoluble particles or particulates, polystyrene particles, i.e., latex particles, are preferably used. Methods, by which an antigen or antibody is supported on the particulates, have been known by those skilled in the art. Of course, the reagent of the present invention may not comprise insoluble carriers.

The reagent for biochemical assays of the present invention may be one that comprises, as essential components, an alginic type polymer, components that should be contained in a liquid for dilution, and an antigen or antibody that should be contained in an agglutination reagent. This reagent for biochemical assays is one that plays as both a liquid for dilution and an agglutination reagent. When this reagent is used, it is unnecessary to preliminary dilute a specimen. By simply mixing the specimen with this reagent, an adequate agglutination reaction may occur.

3. Kit for Biochemical Assay

A kit for a biochemical assay is also provided that comprises the reagent for biochemical assays according to the present invention (hereafter "reagent (x)"), and at least one member selected from the group consisting of a reagent (hereafter "reagent (y)") other than the reagent (x), a container, and an instruction for use.

For example, if the kit comprises a diluent for specimens comprising an alginic type polymer, it may further comprise, as other constitutional elements, a target-positive control sample (for example, this comprises a standard compound for calibration), a target-negative control sample, an agglutination reagent comprising particulate carriers on which an antigen of antibody that is a target to be detected or an antibody against an antigen that is a target to be detected is supported, a reaction container, an instruction for use, etc. In the instruction for use, the amount of a specimen, the amount(s) of the reagent(s), reaction conditions, a method for determination, a method for judging the results, etc. are generally explained.

For example, if the kit comprises an agglutination reagent comprising an alginic type polymer and an antigen or antibody, it may further comprise a liquid for dilution which does not comprise an alginic type polymer. If the kit comprises an agglutination reagent comprising an alginic type polymer, components that should be contained in a liquid for dilution, and particulate carriers on which an antigen or antibody is supported, it may further comprise only an instruction for use.

4. Biochemical Assay Method

The biochemical assay method of the present invention comprises a step of bringing a compound that can specifically bond to a target to be detected into contact with a specimen that may contain the target in the presence of at least one member selected from the group consisting of alginic acid and alginates and a step of determining the amount of agglutinations that have been made by the specific reaction between the compound and the target. When the specific reaction is an antigen-antibody reaction, (1) the compound may be an antibody and the target may be an antigen of the antibody or (2) the compound may be an antigen and the target may be an antibody against the antigen.

In the step for reaction, an agglutination reagent, an alginic type polymer, and a specimen should coexist in a liquid. However, the method by which the liquid is prepared, in other words, the order of the addition or mixing of them, is not limited. Thus, they may be mixed at one time. Or, before the reaction step, the agglutination reagent and the alginic type polymer, the agglutination reagent and the specimen, or the alginic type polymer and the specimen may be mixed. Specifically, for example, a specimen is diluted with a diluent for specimens comprising an alginic type polymer, and then to the diluted specimen thus prepared, an agglutination reagent is added. Or, a suspension comprising an alginic type polymer and an agglutination reagent is prepared, and then to the suspension, an specimen is added.

The reaction temperature is not limited. However, it is in the range of generally 4-50°C, preferably 15-40°C, and more preferably 30-40°C. If the temperature is extremely low, occasions to collide antigens with antibodies may decrease. Also, occasions to collide antigen- or antibody-sensitized particles may decrease. If the temperature is extremely high, the stability of the immune complex comprising an antigen and an antibody against it may decrease.

The reaction time is not limited. However, it is in the range of generally 0-60 minutes, and preferably 1-30 minutes. If the reaction time is extremely short, the immune complex may be insufficiently formed, especially in the case where the concentration of the target to be detected is low in the reaction mixture. If the reaction time is extremely long, the stability of the immune complex may decrease. The step for reaction and the step for determination may overlap.

It is preferable that the reaction is conducted in a buffer solution which is commonly used in the biochemical field. The pH of the reaction mixture is in the range of generally 5-10, and preferably 6-9 from the view point of the stability of the immune complex.

In the step for determination, whether agglutination has been formed may be judged with the naked eye. However, from the view point of the unity of the judgment and to treat a large number of specimens, it is preferable to determine the degree or amount of agglutinations by using an optical determination instrument. The instrument may be one that is based on the principle of nephelometry or one that is based on the principle of turbidimetry.

5. Use of Alginic type polymer as Accelerator for Agglutination Reactions

The alginic type polymer is used to facilitate agglutination reactions. Specifically, in a reaction mixture for an immune agglutination reaction, the alginic type polymer is used. Because of the presence of it, the immune agglutination reaction is facilitated and the sensitivity of the reaction is enhanced. Methods for using the alginic type polymer in various agglutination reactions have been already explained in this specification.

According to the present invention, an accelerator for agglutination reactions can be provided that is suitable to use in various agglutination determination systems (e.g., immune agglutination determination systems), shows a strong accelerative function to agglutination reactions, does not tend to increase the viscosity of its aqueous solution, shows good reproducibility and precision in the determination results, and can be readily handled. By using the accelerator of the present invention, a high sensitivity and a determination or quantitative analysis in a wide range can be realized especially in latex agglutination determination systems.

Further, according to the method of the present invention, the viscosities of reaction mixtures do not tend to increase, and based on agglutination reactions, results of biochemical assays that show good reproducibility and precision can be readily obtained.

Examples

Example 1: Test about Function to Facilitate Immune Agglutination

1. Preparation of Diluents for Specimens

By dissolving sodium dihydrogenphosphate and disodium hydrogenphosphate in deionized water, a phosphate buffer (pH7.4) having a total concentration of phosphates of 0.1M was prepared. Then, by dissolving bovine serum albumin (BSA) in the phosphate buffer in an amount that the concentration of BSA comes to 1 w/v %, 1 % BSA/phosphate buffer hereafter "1 % BSA-PB") was prepared. The 1 % BSA-PB was also called as a diluent for specimens comprising no accelerator (hereafter "diluent No. 0 for specimens"). To diluent No. 0 for specimens, sodium alginate (grade: 300-400 cp; Wako Pure Chemical Industries, Ltd.; viscosity: 300-400 cp [1 w/v %, at 20°C]) was added in an amount that the concentration of sodium alginate comes to 0.080 w/v %. Thus, a diluent for specimens comprising an accelerator of the present invention (hereafter "diluent No. 1 for specimens") was prepared. Further, diluent Nos. 2-10 for specimens, which respectively comprised accelerators listed in Table 1 in amounts listed in Table 1, were prepared in the same way.

**Table 1**

| No. | Accelerator | Manufacturer | Conc. of accelerator in diluent for specimens (w/v) | Final conc. of accelerator in reaction mixture (w/v) |
|---|---|---|---|---|
| 1 | sodium alginate 300~400cp | Wako Pure Chemical Industries, Ltd. | 0.080% | 0.067 % |
| 2 | polyvinylpyrrolidone K-90 (M.W.=1,200,000) | Junsei Chemical Co., Ltd. | 0.700% | 0.583 % |
| 3 | pectin | Wako Pure Chemical Industries, Ltd. | 0.250% | 0.208 % |
| 4 | hydroxyethyl cellulose 1000~4000cp | Wako Pure Chemical Industries, Ltd. | 0.230% | 0.192 % |
| 5 | propylene glycol alginate | Wako Pure Chemical Industries, Ltd. | 0.150% | 0.125 % |
| 6 | FICOLL* (Approx. Mol .Wt.: 400,000) Type400 | SIGMA | 5.000% | 4.167 % |
| 7 | poly(vinylsulfuric acid potassium salt) | Nacalai Tesque, Inc. | 1.150% | 0.958 % |
| 8 | polyacrylic acid 250,000 | Wako Pure Chemical Industries, Ltd. | 0.220% | 0.183 % |
| 9 | carboxymethyl cellulose sodium salt | Wako Pure Chemical Industries, Ltd. | 0.140% | 0.117 % |
| 10 | polyethylene glycol 500,000 (M.W.=300,000~500,000) | Wako Pure Chemical Industries, Ltd. | 0.375% | 0.313 % |

*: a nonionic synthetic polymer of sucrose

EP 1 355 154 A2

2. Test Method

By using diluent Nos. 0-10 for specimens, accelerative functions for forming agglutinations of the accelerators listed in Table 1 were examined in a determination system for an antibody against a syphilis surface antigen.

Specifically, first, 20 microliter of one of specimens (syphilis-negative simulated serum having a concentration of 0 T.U.* and syphilis-positive standard serums having concentrations of 39, 121, 237, and 405 T.U.*; Sekisui Chemical Co., Ltd.) and 350 microliter of one of diluent Nos. 0-10 for specimens were mixed to each other. After 315.5 seconds, 50 microliter of an agglutination reagent in the form of a suspension comprising latex carrier particles on which a syphilis surface antigen had been sensitized (Mediace TPLA latex suspension; Sekisui Chemical Co., Ltd.) was added to prepare a reaction mixture. 79.7 seconds after the agglutination reagent was added, the absorbance (1st absorbance) of the reaction mixture was determined at a wavelength of 700 nm by using a biochemical automatic analyzer (type 7080; Hitachi, Ltd.). Also, 274.5 seconds after the agglutination reagent was added, the absorbance (2nd absorbance) of the reaction mixture was determined in the same way. The difference between the 1st absorbance and the 2nd absorbance was calculated and identified as absorbance of a specimen.

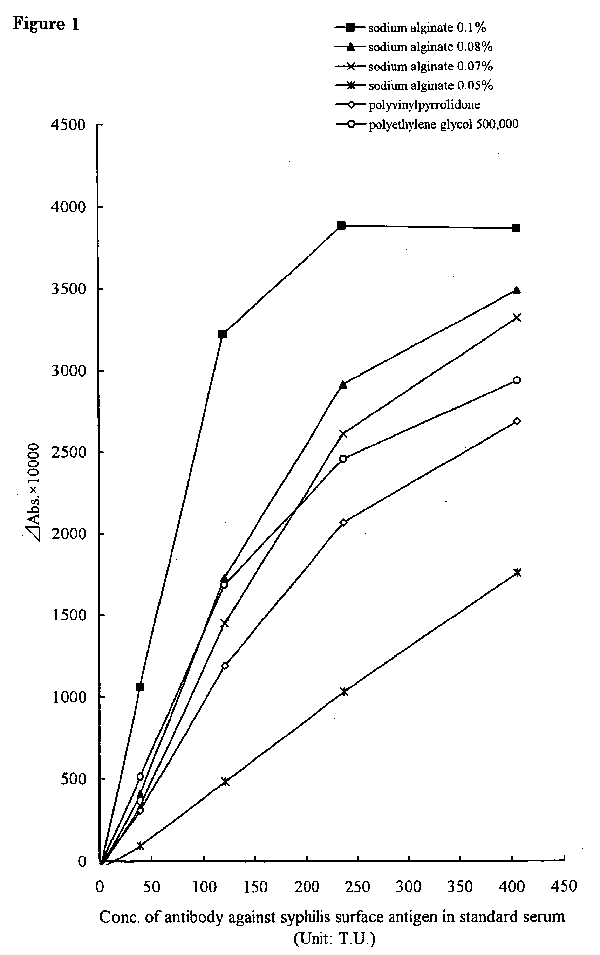

From the results of the determinations, for each accelerator the relationship between the concentrations of antibody (X axis) and the determined values (Y axis) was analyzed. The results are shown in Table 2 and Figure 1. In the column of "blank" in Table 2, the 2nd absorbance of the reaction mixtures containing syphilis-negative simulated serum (0 T.U.) was listed.

Table 2

$\Delta$Abs.$\times$1000

| No. | Accelerator | n | Blank | Conc. of antibody against syphilis surface antigen in standard serum (Unit: T.U.) | | | | |
| --- | --- | --- | --- | --- | --- | --- | --- | --- |
| | | | | 0 | 39 | 121 | 237 | 405 |
| 0 | none | 1 | 10874 | -42 | -34 | 13 | 85 | 198 |
| | | 2 | 10905 | -59 | -11 | 76 | 95 | 195 |
| | | average | 10890 | -51 | -23 | 45 | 90 | 197 |
| 1 | sodium alginate | 1 | 11619 | -43 | 400 | 1730 | 2899 | 3484 |
| | | 2 | 11407 | -52 | 419 | 1733 | 2935 | 3504 |
| | | average | 11513 | -48 | 410 | 1732 | 2917 | 3494 |
| 2 | polyvinylpyrrolidone | 1 | 11427 | -10 | 298 | 1195 | 2058 | 2697 |
| | | 2 | 11390 | -20 | 318 | 1192 | 2081 | 2683 |
| | | average | 11409 | -15 | 308 | 1194 | 2070 | 2690 |
| 3 | pectin | 1 | 11882 | -36 | 379 | 1544 | 2612 | 3209 |
| | | 2 | 11760 | -16 | 385 | 1549 | 2616 | 3228 |
| | | average | 11821 | -26 | 382 | 1547 | 2614 | 3219 |
| 4 | hydroxyethyl hydroxyethyl cellulose | 1 | 11409 | -32 | 319 | 1449 | 2532 | 3180 |
| | | 2 | 11464 | -21 | 333 | 1455 | 2562 | 3175 |
| | | average | 11437 | -27 | 326 | 1452 | 2547 | 3178 |
| 5 | propylene glycol alginate | 1 | 11433 | 4 | 430 | 1749 | 2866 | 3402 |
| | | 2 | 11456 | -11 | 411 | 1757 | 2842 | 3398 |
| | | average | 11445 | -4 | 421 | 1753 | 2854 | 3400 |
| 6 | FICOLL* | 1 | 10949 | -43 | 112 | 473 | 908 | 1330 |
| | | 2 | 10933 | -52 | 93 | 448 | 900 | 1331 |
| | | average | 10941 | -48 | 103 | 461 | 904 | 1331 |

*: a nonionic synthetic polymer of sucrose

*: T.U. = Titer Unit; 1 T.U. = 1.64 ml.U.; A serum having 1,280 titer as a determined value of the TPHAtest has 1,280 T.U..

Table 2   (continued)

| | | | | Conc. of antibody against syphilis surface antigen in standard serum (Unit: T.U.) | | | | ΔAbs.×1000 |
| No. | Accelerator | n | Blank | 0 | 39 | 121 | 237 | 405 |
|---|---|---|---|---|---|---|---|---|
| 7 | poly(vinylsulfiiric acid potassium salt) | 1 | 11433 | -18 | 484 | 1655 | 2322 | 2462 |
| | | 2 | 11362 | -112 | 482 | 1646 | 2187 | 2388 |
| | | average | 11398 | -65 | 483 | 1651 | 2255 | 2425 |
| 8 | polyacrylic acid 250,000 | 1 | 10013 | -25 | 295 | 1135 | 1810 | 2308 |
| | | 2 | 10254 | -50 | 312 | 1166 | 1925 | 2438 |
| | | average | 10134 | -38 | 304 | 1151 | 1868 | 2373 |
| 9 | carboxymethyl cellulose sodium salt | 1 | 10351 | -11 | 358 | 1499 | 2483 | 3064 |
| | | 2 | 10284 | -26 | 377 | 1522 | 2521 | 3116 |
| | | average | 10318 | -19 | 368 | 1511 | 2502 | 3090 |
| 10 | polyethylene glycol 500,000 | 1 | 10608 | -23 | 520 | 1696 | 2470 | 2914 |
| | | 2 | 10769 | -31 | 511 | 1687 | 2452 | 2967 |
| | | average | 10689 | -27 | 516 | 1691 | 2461 | 2941 |

As is apparent from the results, as compared with the case where no accelerator was used (diluent No. 0 for specimens), accelerator Nos. 1-10 had functions to facilitate immune agglutination. However, FICOLL exhibited only a poor effect as compared with other accelerators. Poly(vinylsulfuric acid potassium salt) and polyacrylic acid exhibited low effects in a region where the concentration of the antibody against a syphilis surface antigen was high.

Sodium alginate and propylene glycol alginate, which were accelerators of the present invention, exhibited high effects in the accelerative function throughout the entire regions (i.e., from a low concentration region to a high concentration region). Further, although they were used in extremely low concentrations, they exhibited effects that were equivalent to or higher than those of other accelerators.

Example 2: Study of Concentrations of Sodium Alginate in Diluent for Specimens and in Reaction Mixture

In the same way as described in Example 1, 1 % BSA-PB was prepared. By using this, diluent Nos. 1-6 for specimens listed in Table 3 were prepared. Table 3 also discloses the names of the accelerators and their concentrations.

By using diluent Nos. 1-6 for specimens, an antibody against a syphilis surface antigen was determined in the same way as described in Example 1.

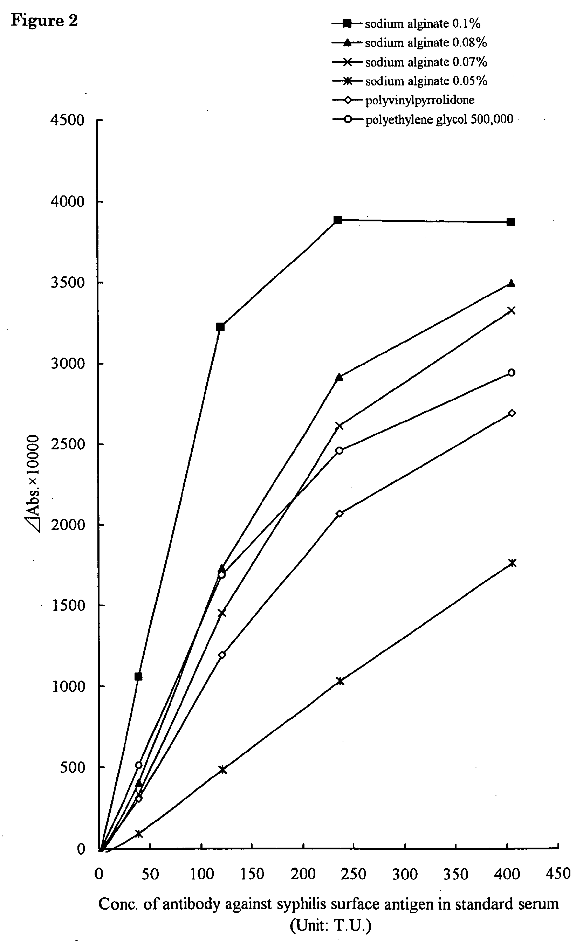

From the results of the determinations, for each accelerator the relationship between the concentrations of antibody (X axis) and the determined values (Y axis) was analyzed. The results are shown in Table 4 and Figure 2.

**Table 3**

| No. | Accelerator | Manufacturer | Conc. of accelerator in diluent for specimens (w/v) | Final conc. of accelerator in reaction mixture (w/v) |
|---|---|---|---|---|
| 1~4 | sodium alginate 300~400cp | Wako Pure Chemical Industries, Ltd. | 0.05~0.1% | 0.042~0.083% |
| 5 | polyvinylpyrrolidone K-90 (M.W.=1,200,000) | Junsei Chemical Co., Ltd. | 0.700% | 0.583% |
| 6 | polyethylene glycol 500,000 (M.W.=300,000~500,000) | Wako Pure Chemical Industries, Ltd. | 0.375% | 0.313% |

**Table 4**

$\triangle$Abs.$\times 10000$

| No. | Accelerator | Conc. of accelerator in diluent for specimens (w/v) | n | Blank | Conc. of antibody against syphilis surface antigen in standard serum (Unit: T.U.) | | | | |
|---|---|---|---|---|---|---|---|---|---|
| | | | | | 0 | 39 | 121 | 237 | 405 |
| 1 | sodium alginate 300~400cp | 0.10% | 1 | 11496 | -56 | 1031 | 3246 | 3866 | 3902 |
| | | | 2 | 11483 | -54 | 1092 | 3207 | 3908 | 3837 |
| | | | average | 11490 | -55 | 1062 | 3227 | 3887 | 3870 |
| 2 | | 0.08% | 1 | 11619 | -43 | 400 | 1730 | 2899 | 3484 |
| | | | 2 | 11407 | -52 | 419 | 1733 | 2935 | 3504 |
| | | | average | 11513 | -48 | 410 | 1732 | 2917 | 3494 |
| 3 | | 0.07% | 1 | 11499 | -46 | 341 | 1452 | 2622 | 3339 |
| | | | 2 | 11427 | -46 | 334 | 1457 | 2607 | 3309 |
| | | | average | 11463 | -46 | 338 | 1455 | 2615 | 3324 |
| 4 | | 0.05% | 1 | 11542 | -44 | 101 | 491 | 1051 | 1748 |
| | | | 2 | 11383 | -42 | 87 | 480 | 1017 | 1777 |
| | | | average | 11463 | -43 | 94 | 486 | 1034 | 1763 |
| 5 | polyvinylpyrrolidone | 0.70% | 1 | 11427 | -10 | 298 | 1195 | 2058 | 2697 |
| | | | 2 | 11390 | -20 | 318 | 1192 | 2081 | 2683 |
| | | | average | 11409 | -15 | 308 | 1194 | 2070 | 2690 |
| 6 | polyethylene glycol 500,000 | 0.375% | 1 | 10608 | -23 | 520 | 1696 | 2470 | 2914 |
| | | | 2 | 10769 | -31 | 511 | 1687 | 2452 | 2967 |
| | | | average | 10689 | -27 | 516 | 1691 | 2461 | 2941 |

EP 1 355 154 A2

As the concentration of sodium alginate increased, the reactivity of the immunoreaction, i.e., the accelerative function, was enhanced. However, when the concentration of sodium alginate in the diluent for specimens was 0.1 w/v %, in a region where the concentration of the antibody was high, the determined values were invariable. Namely, a region where the relationship between the concentrations of antibody (X axis) and the determined values (Y axis) was linear was narrow.

Example 3: Test of Storage Stability

In the same way as described in Example 1, 1 % BSA-PB was prepared. By using this, diluent Nos. 1-8 for specimens listed in Table 5 were prepared. Table 5 also discloses the names of the accelerators and their concentrations.

Diluent Nos. 1-8 for specimens were stored at 40°C for one day or seven days. By using three kinds (i.e., the periods of storage were zero, one day, and seven days) of diluent Nos. 1-8 for specimens, an antibody against a syphilis surface antigen was determined in the same way as described in Example 1.

From the results of the determinations, for each accelerator the relationship between the concentrations of antibody and the determined values was analyzed. Also, for each accelerator the influence of storage at 40°C was analyzed. The results are shown in Table 6.

In Table 6, the differential rate (%) in the column of "Blank" was calculated as follows:

$$\text{Differential rate (\%)} = [(\text{average of the values determined by using the diluent}$$
$$\text{for specimens that was stored one or seven days})/(\text{average of the values determined by}$$
$$\text{using the diluent for specimens that was not stored})] \times 100$$

The differential rate (%) in the column of "Conc. of antibody against syphilis surface antigen in standard serum" was calculated as follows:

$$\text{Differential rate (\%)} = \{[(\text{average of the values determined by using the diluent}$$
$$\text{for specimens that was stored one or seven days})/(\text{average of the values determined by}$$
$$\text{using the diluent for specimens that was not stored})] \times 100\} - 100$$

**Table 5**

| No. | Accelerator | Manufacturer | Conc. of accelerator in diluent for specimens (w/v) | Final conc. of accelerator in reaction mixture (w/v) |
|---|---|---|---|---|
| 1 | sodium alginate 300~400cp | Wako Pure Chemical Industries, Ltd. | 0.080% | 0.067 % |
| 2 | polyvinylpyrrolidone K-90 (M.W.=1,200,000) | Junsei Chemical Co., Ltd. | 0.700% | 0.583 % |
| 3 | pectin | Wako Pure Chemical Industries, Ltd. | 0.250% | 0.208 % |
| 4 | hydroxyethyl cellulose 1000~4000cp | Wako Pure Chemical Industries, Ltd. | 0.230% | 0.192 % |
| 5 | propylene glycol alginate | Wako Pure Chemical Industries, Ltd. | 0.150% | 0.125 % |
| 6 | polyacrylic acid 250,000 | Wako Pure Chemical Industries, Ltd. | 0.220% | 0.183 % |
| 7 | carboxymethyl cellulose sodium salt | Wako Pure Chemical Industries, Ltd. | 0.140% | 0.117 % |
| 8 | polyethylene glycol 500,000 (M.W.=300,000~500,000) | Wako Pure Chemical Industries, Ltd. | 0.375% | 0.313 % |

Table 6 (No.1)

| No | Accelerator | Conc. of accelerator | Number of days stored | Blank | Conc. of antibody against syphilis surface antigen in standard serum (Unit: T.U.) ΔAbs. × 10000 | | | | |
|---|---|---|---|---|---|---|---|---|---|
| . | | | | | 0 | 39 | 121 | 237 | 405 |
| 1 | sodium alginate | 0.080% | 0 | 11456 | -22 | 262 | 1281 | 2347 | 3083 |
| | | | | 11421 | -16 | 269 | 1264 | 2338 | 3085 |
| | | | average | 11439 | -19 | 266 | 1273 | 2343 | 3084 |
| | | | 1 | 11528 | -12 | 295 | 1234 | 2430 | 3117 |
| | | | | 11535 | -20 | 313 | 1344 | 2386 | 3118 |
| | | | average | 11532 | -16 | 304 | 1289 | 2408 | 3118 |
| | | | differential rate (%) | 100.8 | - | 14.5 | 1.3 | 2.8 | 1.1 |
| | | | 7 | 11450 | 17 | 300 | 1327 | 2480 | 3116 |
| | | | | 11533 | 23 | 288 | 1380 | 2361 | 3095 |
| | | | average | 11492 | 20 | 294 | 1354 | 2421 | 3106 |
| | | | differential rate (%) | 100.5 | - | 10.7 | 6.4 | 3.3 | 0.7 |
| 2 | polyvinylpyrrolidone | 0.700% | 0 | 11427 | -10 | 298 | 1195 | 2058 | 2697 |
| | | | | 11390 | -20 | 318 | 1192 | 2081 | 2683 |
| | | | average | 11409 | -15 | 308 | 1194 | 2070 | 2690 |
| | | | 1 | 11508 | -9 | 312 | 1218 | 2089 | 2720 |
| | | | | 11529 | -9 | 321 | 1213 | 2116 | 2726 |
| | | | average | 11519 | -9 | 317 | 1216 | 2103 | 2723 |
| | | | differential rate (%) | 101.0 | - | 2.8 | 1.8 | 1.6 | 1.2 |
| | | | 7 | 11419 | -10 | 328 | 1260 | 2165 | 2739 |
| | | | | 11461 | -2 | 331 | 1260 | 2157 | 2787 |
| | | | average | 11440 | -6 | 330 | 1260 | 2161 | 2763 |
| | | | differential rate (%) | 100.3 | - | 7.0 | 5.6 | 4.4 | 2.7 |

Table 6 (No.1) (continued)

| No | Accelerator | Conc. of accelerator | Number of days stored | Blank | Conc. of antibody against syphilis surface antigen in standard serum (Unit: T.U.) $\Delta$Abs. $\times$ 10000 | | | | |
|---|---|---|---|---|---|---|---|---|---|
| | | | | | 0 | 39 | 121 | 237 | 405 |
| 3 | pectin | 0.250% | 0 | 11777 | -3 | 413 | 1629 | 2652 | 3225 |
| | | | | 11766 | -19 | 381 | 1581 | 2694 | 3207 |
| | | | average | 11772 | -11 | 397 | 1605 | 2673 | 3216 |
| | | | 1 | 11786 | -21 | 288 | 1137 | 2069 | 2825 |
| | | | | 11776 | -26 | 276 | 1112 | 2088 | 2813 |
| | | | average | 11781 | -24 | 282 | 1125 | 2079 | 2819 |
| | | | differential rate (%) | 100.1 | - | -29.0 | -29.9 | -22.2 | -12.3 |
| | | | 7 | 11740 | -17 | 82 | 376 | 821 | 1381 |
| | | | | 11725 | -32 | 87 | 395 | 831 | 1367 |
| | | | average | 11733 | -25 | 85 | 386 | 826 | 1374 |
| | | | differential rate (%) | 99.7 | - | -78.7 | -76.0 | -69.1 | -57.3 |
| 4 | hydroxyethyl cellulose | 0.230% | 0 | 11409 | -32 | 319 | 1449 | 2532 | 3180 |
| | | | | 11464 | -21 | 333 | 1455 | 2562 | 3175 |
| | | | average | 11437 | -27 | 326 | 1452 | 2547 | 3178 |
| | | | 1 | 11552 | -7 | 358 | 1384 | 2465 | 3147 |
| | | | | 11476 | -5 | 344 | 1391 | 2476 | 3170 |
| | | | average | 11514 | -6 | 351 | 1388 | 2471 | 3159 |
| | | | differential rate (%) | 100.7 | - | 7.7 | -4.4 | -3.0 | -0.6 |
| | | | 7 | 11474 | -29 | 253 | 1026 | 1911 | 2680 |
| | | | | 11486 | -12 | 242 | 1000 | 1929 | 2718 |
| | | | average | 11480 | -21 | 248 | 1013 | 1920 | 2699 |
| | | | differential rate (%) | 100.4 | - | -24.1 | -30.2 | -24.6 | -15.1 |

Table 6 (No.2)

| | | | | | ΔAbs.×10000 | | | | |
|---|---|---|---|---|---|---|---|---|---|
| No | Accelerator | Conc. of accelerator | Number of days stored | Blank | Conc. of antibody against syphilis surface antigen in standard serum (Unit: T.U.) | | | | |
| . | | | | | 0 | 39 | 121 | 237 | 405 |
| 5 | propylene glycol alginate | 0.150% | 0 | 11562 | -16 | 262 | 1163 | 2212 | 2996 |
| | | | | 11615 | -6 | 231 | 1158 | 2177 | 3029 |
| | | | average | 11589 | -11 | 247 | 1161 | 2195 | 3013 |
| | | | 1 | 11594 | -38 | 95 | 415 | 884 | 1447 |
| | | | | 11423 | -43 | 81 | 401 | 847 | 1440 |
| | | | average | 11509 | -41 | 88 | 408 | 866 | 1444 |
| | | | differential rate (%) | 99.3 | - | -64.3 | -64.8 | -60.6 | -52.1 |
| 6 | polyacrylic acid 250,000 | 0.22% | 0 | 10013 | -25 | 295 | 1135 | 1810 | 2308 |
| | | | | 10254 | -50 | 312 | 1166 | 1925 | 2438 |
| | | | average | 10134 | -38 | 304 | 1151 | 1868 | 2373 |
| | | | 7 7 | 10110 9969 | 10 -6 | 299 306 | 1091 1098 | 1861 1862 | 2290 2348 |
| | | | average | 10040 | 2 | 303 | 1095 | 1862 | 2319 |
| | | | differential rate (%) | 99.1 | - | -0.3 | -4.9 | -0.3 | -2.3 |
| 7 | carboxymethyl cellulose sodium salt | 0.14% | 0 | 11228 11186 | -35 -10 | 483 433 | 1869 1900 | 2965 3011 | 3620 3536 |
| | | | average | 11207 | -23 | 458 | 1885 | 2988 | 3578 |
| | | | 1 | 11267 11347 | -22 -4 | 420 423 | 1566 1541 | 2781 2646 | 3323 3362 |
| | | | average | 11307 | -13 | 422 | 1554 | 2714 | 3343 |
| | | | differential rate (%) | 100.9 | - | -8.0 | -17.6 | -9.2 | -6.6 |
| | | | 7 | 9564 10148 | -31 -43 | 238 246 | 998 978 | 1916 1907 | 2621 2509 |
| | | | average | 9856 | -37 | 242 | 988 | 1912 | 2565 |
| | | | differential rate (%) | 87.9 | - | -47.2 | -47.6 | -36.0 | -28.3 |

Table 6 (No.2)   (continued)

| | | | | | $\Delta$Abs.$\times$10000 | | | | |
|---|---|---|---|---|---|---|---|---|---|
| No | Accelerator | Conc. of accelerator | Number of days stored | Blank | Conc. of antibody against syphilis surface antigen in standard serum (Unit: T.U.) | | | | |
| . | | | | | 0 | 39 | 121 | 237 | 405 |
| 8 | polyethylene glycol 50000 | 0.375% | 0 | 10344 10304 | 3 -11 | 399 402 | 1384 1382 | 2153 2086 | 2504 2687 |
| | | | average | 10324 | -4 | 401 | 1383 | 2120 | 2596 |
| | | | 1 | 10340 10425 | -6 -15 | 369 350 | 1262 1251 | 1988 2022 | 2616 2568 |
| | | | average | 10383 | -11 | 359 | 1257 | 2005 | 2592 |
| | | | differential rate (%) | 100.6 | - | -10.3 | -9.1 | -5.4 | -0.1 |
| | | | 7 | 10119 10247 | -40 -19 | 245 291 | 975 961 | 1691 1765 | 2181 2293 |
| | | | average | 10183 | -30 | 268 | 968 | 1728 | 2237 |
| | | | differential rate (%) | 98.6 | - | -33.2 | -30.0 | -18.5 | -13.8 |

The diluent for specimens comprising sodium alginate, polyacrylic acid, or polyvinylpyrrolidone was stable after it had been stored at 40°C for seven days. Namely, its function for facilitating immune agglutination reactions was not decreased. About accelerators other than sodium alginate, polyacrylic acid, and polyvinylpyrrolidone, their functions for facilitating immune agglutination reactions were decreased by storing at 40°C.

Example 4: Comparison of Reproducibility

In the same way as described in Example 1, 1 % BSA-PB was prepared. By using this, diluent Nos: 1-5 for specimens listed in Table 7 were prepared. Table 7 also discloses the names of the accelerators and their concentrations.

By using diluent Nos. 1-5 for specimens, syphilis-negative simulated serum, and syphilis-positive standard serums, an antibody against a syphilis surface antigen was determined in the same way as described in Example 1. From the results of the determinations, for each accelerator the relationship between the concentrations of antibody and the determined values was analyzed, namely, a calibration curve was made. Then, by using each of diluent Nos. 1-5 for specimens, the concentration of syphilis-antibody in a syphilis-positive serum having a concentration of about 18.6 T.U. as a specimen was determined for ten times. From the results of the determinations, average values, standard deviations, coefficients of variation (CV values), and the like were calculated.

The results are shown in Table 8.

**Table 7**

| No. | Accelerator | Manufacturer | Conc. of accelerator in diluent for specimens (w/v) | Final conc. of accelerator in reaction mixture (w/v) |
|---|---|---|---|---|
| 1 | sodium alginate 300~400cp | Wako Pure Chemical Industries, Ltd. | 0.080% | 0.067 % |
| 2 | polyvinylpyrrolidone K-90   (M.W.=1,200,000) | Junsei Chemical Co., Ltd. | 0.700% | 0.583 % |
| 3 | polyacrylic acid 250,000 | Wako Pure Chemical Industries, Ltd. | 0.220% | 0.183 % |
| 4 | carboxymethyl cellulose sodium salt | Wako Pure Chemical Industries, Ltd. | 0.140% | 0.117 % |
| 5 | polyethylene glycol 500,000   (M.W.=300,000~500,000) | Wako Pure Chemical Industries, Ltd. | 0.375% | 0.313 % |

**Table 8**

| n | sodium alginate 0.080% | polyvinylpyrrolidone 0.700% | polyacrylic acid 0.220% | carboxymethyl cellulose sodium salt 0.140% | polyethylene glycol 0.375% |
|---|---|---|---|---|---|
| 1 | 20.0 | 19.8 | 24.9 | 14.5 | 16.9 |
| 2 | 19.0 | 18.0 | 18.8 | 14.0 | 16.8 |
| 3 | 20.1 | 18.0 | 17.4 | 15.4 | 18.5 |
| 4 | 21.6 | 19.2 | 16.4 | 13.1 | 17.8 |
| 5 | 19.9 | 21.1 | 17.1 | 14.2 | 15.5 |
| 6 | 20.3 | 21.2 | 20.0 | 15.3 | 18.6 |
| 7 | 20.3 | 19.2 | 18.2 | 16.0 | 17.1 |
| 8 | 20.2 | 17.6 | 17.7 | 15.8 | 19.2 |
| 9 | 19.3 | 20.2 | 18.3 | 15.9 | 19.7 |
| 10 | 19.4 | 19.8 | 20.6 | 16.2 | 18.6 |
| average | 20.0 | 19.4 | 18.9 | 15.0 | 17.9 |
| standard deviation (S.D.) | 0.7 | 1.3 | 2.5 | 1.0 | 1.3 |
| coefficient of variation (CV(%)) | 3.6% | 6.5% | 12.9% | 6.9% | 7.2% |
| average + S.D. | 20.7 | 20.7 | 21.4 | 16.1 | 19.2 |
| average - S.D. | 19.3 | 18.1 | 16.5 | 14.0 | 16.6 |
| maximun value | 21.6 | 21.2 | 24.9 | 16.2 | 19.7 |
| minimun value | 19.0 | 17.6 | 16.4 | 13.1 | 15.5 |
| max. - min. | 2.6 | 3.6 | 8.5 | 3.1 | 4.2 |

EP 1 355 154 A2

From the results shown in Table 8, it was understood that sodium alginate showed a less CV value than those of other accelerators. Namely, sodium alginate showed good reproducibility.

Example 5: Measurement of Viscosity

In the same way as described in Example 1, 1 % BSA-PB was prepared. By using this, diluent Nos. 1-5 for specimens listed in Table 9 were prepared. Table 9 also discloses the names of the accelerators and their concentrations.

The viscosities of diluent Nos. 1-5 for specimens were measured at a room temperature (about 24°C) by using an Ostwald viscometer (relative viscometer; TOP-K-11212-18; Sogo Rikagaku Glass Seisakusho). Specifically, 5 milliliter of a sample (a diluent for specimens) having a temperature of 24°C was injected into an inside of the Ostwald viscometer by using a pipet. This sample was sucked up from one end of a tube of the Ostwald viscometer to a certain height and then flowed down by itself. The time (seconds) that was required to flow down in a definite interval of the tube was measured.

The results are shown in Table 10.

**Table 9**

| No. | Accelerator | Manufacturer | Conc. of accelerator in diluent for specimens (w/v) | Final conc. of accelerator in reaction mixture (w/v) |
|---|---|---|---|---|
| 1 | sodium alginate 300~400cp | Wako Pure Chemical Industries, Ltd. | 0.080% | 0.067 % |
| 2 | polyvinylpyrrolidone K-90 (M.W.=1,200,000) | Junsei Chemical Co., Ltd. | 0.700% | 0.583 % |
| 3 | polyacrylic acid 250,000 | Wako Pure Chemical Industries, Ltd. | 0.220% | 0.183 % |
| 4 | carboxymethyl cellulose sodium salt | Wako Pure Chemical Industries, Ltd. | 0.140% | 0.117 % |
| 5 | polyethylene glycol 500,000 (M.W.=300,000~500,000) | Wako Pure Chemical Industries, Ltd. | 0.375% | 0.313 % |

EP 1 355 154 A2

Table 10

| Accelerator | Conc. of accelerator in diluent for specimens (w/v) | n | Time required for dropping (sec.) | Average |
|---|---|---|---|---|
| sodium alginate | 0.080% | 1 | 375.0 | 375 |
| | | 2 | 375.0 | |
| polyvinylpyrrolidone | 0.700% | 1 | 394.0 | 395 |
| | | 2 | 395.0 | |
| polyacrylic acid | 0.220% | 1 | 619 | 618 |
| | | 2 | 617 | |
| carboxymethyl cellulose sodium salt | 0.140% | 1 | 374 | 376 |
| | | 2 | 377 | |
| polyethylene glycol | 0.375% | 1 | 404 | 405 |
| | | 2 | 405 | |

The diluent for specimens comprising sodium alginate or carboxymethyl cellulose sodium salt in a concentration that can show accelerative function for forming agglutinations had a lower viscosity than other diluents for specimens comprising accelerators in concentrations that can show accelerative function for forming agglutinations. Thus, the diluent for specimens comprising sodium alginate and the diluent for specimens comprising carboxymethyl cellulose sodium salt were preferable.

Example 6: Comparison among Sodium Alginates of Different Grades

In the same way as described in Example 1, 1 % BSA-PB was prepared. By using this, diluent Nos. 1-4 for specimens listed in Table 11 were prepared. Table 11 also discloses the names and the grades (i.e., types) of the accelerators, and their concentrations.
In the same way as described in Example 5, the viscosities of diluent Nos. 1-4 for specimens were measured.
In the same way as described in Example 4, for each accelerator the relationship between the concentrations of antibody and the determined values was analyzed, namely, a calibration curve was made. Then, the concentration of syphilis-antibody in a syphilis-positive serum having a concentration of about 18.6 T.U. as a specimen was determined for ten times. From the results of the determinations, average values, standard deviations, coefficients of variation (CV values), and the like were calculated.
The results are shown in Tables 12-14.

Table 11

| No. | Accelerator | Type* | Manufacturer | Conc. of accelerator in diluent for specimens (w/v) | Final conc. of accelerator in reaction mixture (w/v) |
|---|---|---|---|---|---|
| 1 | polyvinylpyrrolidone K-90 (M.W.=1,200,000) | | Junsei Chemical Co., Ltd. | 0.700% | 0.583 % |
| 2 | sodium alginate | 100~150cp | Wako Pure Chemical Industries, Ltd. | 0.090% | 0.075 % |
| 3 | sodium alginate | 300~400cp | Wako Pure Chemical Industries, Ltd. | 0.080% | 0.067 % |
| 4 | sodium alginate | 500~600cp | Wako Pure Chemical Industries, Ltd. | 0.075% | 0.063 % |

*: This corresponds to the viscosity of 1 w/v % aqueous solution at 20°C.

Table 12

| Accelerator | | PVP | sodium alginate | | |
|---|---|---|---|---|---|
| Type | | | 100~150cp | 300~400cp | 500~600cp |
| Conc. of accelerator in diluent for specimens (w/v) | | 0.70% | 0.09% | 0.08% | 0.075% |
| n | 1 | 394.0 | 345.0 | 375.0 | 406.0 |
| | 2 | 395.0 | 347.0 | 375.0 | 407.0 |
| average | | 394.5 | 346.0 | 375.0 | 406.5 |

Unit: sec.

EP 1 355 154 A2

**Table 13**

$\triangle$Abs.×10000

| Accelerator | Type | Conc. of accelerator in diluent for specimens (w/v) | n | Blank | Conc. of antibody against syphilis surface antigen in standard serum (Unit: T.U.) | | | | |
|---|---|---|---|---|---|---|---|---|---|
| | | | | | 0 | 39 | 121 | 237 | 405 |
| PVP | | 0.70% | 1 | 10607 | -7 | 384 | 1456 | 2402 | 2938 |
| | | | 2 | 10828 | -12 | 361 | 1504 | 2449 | 2986 |
| | | | average | 10718 | -10 | 373 | 1480 | 2426 | 2962 |
| sodium alginate | 100~150cp | 0.09% | 1 | 10881 | -21 | 352 | 1541 | 2631 | 3363 |
| | | | 2 | 10879 | -8 | 369 | 1521 | 2620 | 3356 |
| | | | average | 10880 | -15 | 361 | 1531 | 2626 | 3360 |
| | 300~400cp | 0.08% | 1 | 10730 | -29 | 377 | 1574 | 2800 | 3444 |
| | | | 2 | 10702 | -13 | 388 | 1570 | 2707 | 3459 |
| | | | average | 10716 | -21 | 383 | 1572 | 2754 | 3452 |
| | 500~600cp | 0.075% | 1 | 10798 | -14 | 382 | 1613 | 2730 | 3431 |
| | | | 2 | 10658 | -30 | 388 | 1533 | 2750 | 3506 |
| | | | average | 10728 | -22 | 385 | 1573 | 2740 | 3469 |

Table 14

| Accelerator | | PVP | sodium alginate | | |
|---|---|---|---|---|---|
| Type | | | 100~150cp | 300~400cp | 500~600cp |
| Conc. of accelerator in diluent for specimens (w/v) | | 0.70% | 0.09% | 0.08% | 0.075% |
| n | 1 | 18.9 | 15.2 | 17.2 | 19.4 |
| | 2 | 18.4 | 17.0 | 16.4 | 17.3 |
| | 3 | 16.9 | 17.2 | 14.0 | 17.6 |
| | 4 | 15.5 | 16.4 | 18.0 | 15.2 |
| | 5 | 17.5 | 16.7 | 18.0 | 16.4 |
| | 6 | 19.9 | 16.8 | 18.0 | 19.0 |
| | 7 | 18.5 | 17.0 | 15.7 | 17.3 |
| | 8 | 17.8 | 17.4 | 16.5 | 15.0 |
| | 9 | 16.9 | 16.6 | 16.0 | 14.8 |
| | 10 | 17.8 | 16.6 | 17.1 | 17.2 |
| average | | 17.8 | 16.7 | 16.7 | 16.9 |
| standard deviation (S.D.) | | 1.2 | 0.6 | 1.3 | 1.6 |
| coefficient of variation (CV(%)) | | 6.9% | 3.6% | 7.6% | 9.4% |
| average + S.D. | | 19.0 | 17.3 | 18.0 | 18.5 |
| average - S.D. | | 16.6 | 16.1 | 15.4 | 15.3 |
| maximun value | | 19.9 | 17.4 | 18.0 | 19.4 |
| minimun value | | 15.5 | 15.2 | 14.0 | 14.8 |
| max. - min. | | 4.4 | 2.2 | 4.0 | 4.6 |

Unit: T.U.

All grades of sodium alginate tested showed sufficient functions for facilitating immune agglutination reactions. Among them sodium alginate of type 100-150 cp had the lowest viscosity and the best reproducibility, and thus it was especially preferable.

Example 7: Effect in Determination System for HBs Antigen

In the same way as described in Example 1, 1 % BSA-PB was prepared. By using this, diluent Nos. 1-8 for specimens listed in Table 15 were prepared. Table 15 also discloses the names of the accelerators and their concentrations.

Diluent Nos. 1-8 for specimens were examined about their accelerative functions in a determination system for an HBs antigen, i.e., a hepatitis B surface antigen.

Specifically, first, 20 microliter of one of specimens (i.e., a solution comprising no HBs antigen standard compound, and solutions comprising an HBs antigen standard compound at concentrations of 9.6, 48.2, 96.4, and 482.2 IU/ml; Kyokuto Pharmaceutical Industrial Co., Ltd.) and 140 microliter of one of diluent Nos. 1-8 for specimens were mixed to each other. After 315.5 seconds, 140 microliter of an agglutination reagent in the form of a suspension comprising latex carrier particles on which an HBs antibody had been sensitized (Lanpia latex HBs antibody latex suspension; Kyokuto Pharmaceutical Industrial Co., Ltd.) was added to prepare reaction mixtures. 79.7 seconds after the agglutination reagent was added, the absorbance (1st absorbance) of the reaction mixture was determined at a wavelength of 800 nm by using a biochemical automatic analyzer (type 7080; Hitachi, Ltd.). Also, 274.5 seconds after the agglutination reagent was added, the absorbance (2nd absorbance) of the reaction mixture was determined in the same way. The difference between the 1st absorbance and the 2nd absorbance was calculated and identified as absorbance of a specimen.

From the results of the determinations, for each accelerator the relationship between the concentrations of antigen and the determined values was analyzed. The results are shown in Table 16. In the column of "blank" in Table 16, the 2nd absorbance of the reaction mixtures containing the solution comprising no HBs antigen standard compound was listed.

**Table 15**

| No. | Accelerator | Manufacturer | Conc. of accelerator in diluent for specimens (w/v) | Final conc. of accelerator in reaction mixture (w/v) |
|---|---|---|---|---|
| 1~6 | sodium alginate 300~400cp | Wako Pure Chemical Industries, Ltd. | 0.1~0.2% | 0.047~0.093% |
| 7 | polyvinylpyrrolidone K-90 (M.W.=1,200,000) | Junsei Chemical Co., Ltd. | 1.200% | 0.560% |
| 8 | polyethylene glycol 6000 | Wako Pure Chemical Industries, Ltd. | 2.000% | 0.93% |

**Table 16**

<div align="right">△Abs.×10000</div>

| No. | Accelerator | Conc. of accelerator in diluent for specimens (w/v) | n | Blank | Conc. of HBs antigen in standard solution (Unit: IU/ml) | | | | |
|---|---|---|---|---|---|---|---|---|---|
| | | | | | 0.0 | 9.6 | 48.2 | 96.4 | 482.2 |
| 1 | | 0.20% | 1 | 18540 | 151 | 302 | 932 | 1769 | 5514 |
| | | | 2 | 18574 | 175 | 294 | 934 | 1748 | 5504 |
| | | | average | 18557 | 163 | 298 | 933 | 1759 | 5509 |
| 2 | | 0.18% | 1 | 18266 | 20 | 88 | 527 | 1119 | 4830 |
| | | | 2 | 18273 | 6 | 110 | 499 | 1118 | 4838 |
| | | | average | 18270 | 13 | 99 | 513 | 1119 | 4834 |
| 3 | | 0.16% | 1 | 18138 | -47 | 15 | 362 | 772 | 3996 |
| | sodium alginate | | 2 | 18148 | -48 | 57 | 320 | 785 | 4011 |
| | 300~400cp | | average | 18143 | -48 | 36 | 341 | 779 | 4004 |
| 4 | | 0.14% | 1 | 18089 | -28 | 49 | 289 | 606 | 3146 |
| | | | 2 | 18084 | -12 | 26 | 286 | 594 | 3154 |
| | | | average | 18087 | -20 | 38 | 288 | 600 | 3150 |
| 5 | | 0.12% | 1 | 18096 | -6 | 47 | 228 | 470 | 2379 |
| | | | 2 | 18133 | -13 | 37 | 214 | 471 | 2407 |
| | | | average | 18115 | -10 | 42 | 221 | 471 | 2393 |
| 6 | | 0.10% | 1 | 18019 | -14 | 28 | 179 | 356 | 1760 |
| | | | 2 | 18094 | -4 | 46 | 175 | 339 | 1748 |
| | | | average | 18057 | -9 | 37 | 177 | 348 | 1754 |
| 7 | polyvinylpyrrolidone K-90 (M.W.=1,200,000) | 1.20% | 1 | 18778 | 57 | 102 | 512 | 1042 | 4534 |
| | | | 2 | 18637 | 42 | 120 | 483 | 1055 | 4555 |
| | | | average | 18708 | 50 | 111 | 498 | 1049 | 4545 |
| 8 | polyethylene glycol 6000 | 2.00% | 1 | 17550 | -9 | 19 | 125 | 312 | 665 |
| | | | 2 | 17268 | -9 | 25 | 170 | 309 | 686 |
| | | | average | 17409 | -9 | 23 | 153 | 323 | 701 |

Also in this determination system, sodium alginate showed an excellent function for facilitating immune agglutination reactions. The sensitivity of the immune agglutination reaction was enhanced as the concentration of sodium alginate became higher.

This specification involves the above explanations and the entire description of Japanese Patent Application No. 2002-98989 by reference. However, the present invention is defined or limited only by the following claims.

**Claims**

1. An accelerator for agglutination reactions consisting essentially of at least one member selected from the group consisting of alginic acid and alginates.

2. A reagent for biochemical assays comprising at least one member selected from the group consisting of alginic acid and alginates.

3. The reagent according to claim 2, wherein the biochemical assays are immunoassays.

4. The reagent for biochemical assays according to claim 2 or 3, wherein the reagent is a diluent for specimens.

5. The reagent for biochemical assays according to claim 2, which further comprises a compound that can specifically bond to a target to be detected.

6. The reagent for biochemical assays according to claim 5, wherein (1) the compound is an antibody and the target is an antigen of the antibody or (2) the compound is an antigen and the target is an antibody against the antigen.

7. The reagent for biochemical assays according to claim 5, which further comprises carriers on which the compound is supported.

8. The reagent for biochemical assays according to claim 3, wherein the reagent is used in an immune agglutination reaction.

9. A kit for a biochemical assay comprising a reagent (x) according to claim 2, and at least one member selected from the group consisting of a reagent (y) other than the reagent (x), a container, and an instruction for use.

10. The kit according to claim 9, wherein the biochemical assay is an immunoassay.

11. A biochemical assay method comprising a step of bringing a compound that can specifically bond to a target to be detected into contact with a specimen that may contain the target in the presence of at least one member selected from the group consisting of alginic acid and alginates and a step of determining the amount of agglutinations that have been made by the specific reaction between the compound and the target.

12. The biochemical assay method according to claim 11, wherein (1) the compound is an antibody and the target is an antigen of the antibody or (2) the compound is an antigen and the target is an antibody against the antigen, and the specific reaction is an antigen-antibody reaction.

13. Use of at least one member selected from the group consisting of alginic acid and alginates as an accelerator for agglutination reactions.

**Figure 1**

Legend:
- ■— sodium alginate 0.1%
- ▲— sodium alginate 0.08%
- ✕— sodium alginate 0.07%
- ✶— sodium alginate 0.05%
- ◇— polyvinylpyrrolidone
- ○— polyethylene glycol 500,000

Y-axis: △Abs. ×10000

X-axis: Conc. of antibody against syphilis surface antigen in standard serum
(Unit: T.U.)

**Figure 2**